# EUROPÄISCHE PATENTSCHRIFT

(11) **EP 4 290 235 B1**
(45) Veröffentlichungstag und Bekanntmachung des Hinweises auf die Patenterteilung: **23.09.2026**
(21) Anmeldenummer: 22178270.9
(22) Anmeldetag: 10.06.2022
(51) Int. Cl.: G01N 33/543, G01N 33/53

(54) **VERFAHREN ZUR NEUTRALISATION EINER BIOTININTERFERENZ IN BINDUNGSTESTEN**
METHOD FOR NEUTRALIZING A BIOTIN INTERFERENCE IN BINDING ASSAYS
PROCÉDÉ DE NEUTRALISATION D'UNE INTERFÉRENCE DE LA BIOTINE LORS DES ESSAIS DE LIAISON

(43) Veröffentlichungstag der Anmeldung: 13.12.2023
(73) Patentinhaber: Siemens Healthcare Diagnostics Products GmbH, 35041 Marburg (DE)
(72) Erfinder: Balouschek, Carolin, 35091 Cölbe (DE); Schwarz, Herbert, 35102 Lohra (DE); Zander, Norbert, 35039 Marburg (DE); Ehm, Matthias, 35094 Lahntal/ Sterzhausen (DE)
(74) Vertreter: Siemens Healthineers Patent Attorneys

(56) Entgegenhaltungen:
- WO-A1-95/23801
- WO-A1-98/09167
- DE-A1- 102010 050 644
- SPONCHIA G. ET AL: "Mesoporous silica nanoparticles with tunable pore size for tailored gold nanoparticles", vol. 16, no. 2, 16 January 2014 (2014-01-16), Dordrecht, XP055977698, ISSN: 1388-0764, Retrieved from the Internet <URL:http://link.springer.com/article/10.1007/s11051-014-2245-1/fulltext.html> DOI: 10.1007/s11051-014-2245-1
- BUDNYAK T M ET AL: "Preparation and properties of organomineral adsorbent obtained by sol-gel technology", JOURNAL OF THERMAL ANALYSIS AND CALORIMETRY, KLUWER, DORDRECHT, NL, vol. 125, no. 3, 21 June 2016 (2016-06-21), pages 1335 - 1351, XP036031110, ISSN: 1388-6150, [retrieved on 20160621], DOI: 10.1007/S10973-016-5581-9

## Beschreibung

Die vorliegende Erfindung betrifft ein Verfahren zur Neutralisation einer Biotininterferenz in einem Bindungstest zum Nachweis eines Analyten in einer Probe, wobei die Probe mit einer adsorbierenden Substanz in Kontakt gebracht wird.

Biotin, auch als Vitamin B7 oder Vitamin H bezeichnet, ist ein wasserlösliches Vitamin, das eine bedeutende Rolle im Stoffwechsel von Fettsäuren, Kohlenhydraten und Proteinen sowie bei der epigenetischen Genregulation spielt. Der Organismus des Menschen kann Biotin nicht selbst synthetisieren und ist abhängig von der Aufnahme durch die Nahrung. Laut der Deutschen Gesellschaft für Ernährung wird die angemessene Zufuhr von Biotin für Jugendliche und Erwachsene, einschließlich schwangerer und stillender Frauen, auf 30 bis 60 Mikrogramm pro Tag geschätzt. Auf dem Markt gibt es zahlreiche Nahrungsergänzungsmittel, die den oben genannten Tagesbedarf an Biotin abdecken, aber auch solche, die in einem deutlich höheren Dosierungsbereich von beispielsweise zehn Milligramm pro Tag liegen. Allgemein werden Biotin-haltige Nahrungsergänzungsmittel für die Unterstützung des Stoffwechsels und der neurologischen Funktion sowie zur Förderung gesunder Haut, Haare und Nägel genutzt.

Der Einsatz von Biotin reicht jedoch weit über die genannten therapeutischen und kosmetischen Zwecke hinaus. Die hochselektive und stabile Interaktion von Biotin und Avidin bzw. Streptavidin hat eine breite Anwendung in biotechnologischen und labormedizinischen Methoden gefunden und wird beispielsweise seit Jahrzenten in den verschiedensten biochemischen, immunologischen und molekularbiologischen Methoden, einschließlich empfindlicher und präziser Bindungsteste verwendet.

Aufgrund ihrer bioanalytischen Leistungsfähigkeit sind in vitro-diagnostische Bindungsteste in klinischen Laboren weit verbreitet. Ihre vollständige Automatisierbarkeit bietet eine Reihe von Vorteilen, wie z.B. die schnelle Durchführung der Tests sowie eine hohe Empfindlichkeit und Präzision. In Bindungstesten agieren Analyt-spezifische Bindungspartner, die spezifisch mit dem zu bestimmenden Analyten interagieren, als Nachweismoleküle. Typische Bindungsteste sind Immunteste, bei denen die Erkennung und der Nachweis eines Analyten durch eine Antigen-Antikörper-Bindung erfolgt. Je nach Konfiguration des Tests können sowohl Antigen als auch Antikörper der nachzuweisende Analyt sein. In derartigen Immunassays wird das Streptavidin-Biotin-System beispielsweise zu Zwecken der Anreicherung, Abtrennung oder Signalverstärkung eingesetzt. Zum Beispiel können Streptavidin-beschichtete magnetische Partikel an einer Festphase als Mittel zur spezifischen Bindung von biotinylierten Antikörpern, an die der nachzuweisende Analyt gebunden ist, verwendet werden, um den Analyten aus der Flüssigphase anzureichern und anschließend nachzuweisen.

Eine erhöhte Biotin-Konzentration in der Probe kann bei der Durchführung solcher Bindungsteste, die eine Biotin-Streptavidin-Interaktion umfassen, signifikante Störeffekte auslösen, da das probenintrinsische Biotin mit biotinylierten Testkomponenten um Bindungsstellen an Streptavidin konkurriert. In Sandwich-Testformaten ist die gemessene Signalintensität typischerweise direkt proportional zur Analytkonzentration. Probenintrinsisches Biotin konkurriert mit den biotinylierten Bindungspartnern um die Bindung an Streptavidin-gekoppelte Signalkomponenten, schwächt so die Signalintensität und produziert schließlich falsch niedrige Testergebnisse. In kompetitiven Testformaten ist die Signalintensität typischerweise umgekehrt proportional zur Analytkonzentration. Auch hier beeinflusst probenintrinsisches Biotin die Signalintensität und resultiert in einem falsch erhöhten Testergebnis. Im Allgemeinen führen Biotininterferenzen zu falsch niedrigen Ergebnissen hinsichtlich der Analytkonzentration.

Bei Laboruntersuchungen von Patienten, die Biotin einnehmen, können je nach Testformat also falsch erhöhte oder falsch niedrige Analytkonzentrationen gemessen werden, wenn die angewandte Methode auf einem Streptavidin-Biotin-Testprinzip beruht. Unerkannte, durch Biotin verursachte Störungen von Laboruntersuchungen stellen damit ein hohes Risiko bezüglich verzögerter Diagnosestellungen, falscher Diagnosen und unnötiger Behandlungen dar.

Aus dem Stand der Technik sind Verfahren zur Reduzierung der Biotininterferenz bekannt, die eine Vorbehandlung der Probe mit Streptavidin-beschichteten magnetischen Kügelchen (Yang et al., 2020, Clinica Chimica Acta, 505, 130-135) oder mit vernetztem Avidin oder Streptavidin (WO 95/23801 A1) vorsehen oder auf die Verwendung von mit Streptavidin versehenen Mikropartikeln abstellen (Bowen et al., 2019, Clinical Biochemistry, 74, 1-11). In diesen Ansätzen ist typischerweise eine längere Inkubation und eine anschließende, zeitaufwändige Abtrennung der Fängereinheiten, z.B. mittels Zentrifugation notwendig, um weitere Testschritte durchführen zu können.

Demnach sind Verfahren zur Neutralisation einer Biotininterferenz in Biotin-Streptavidin-basierten Bindungstesten bekannt, bei denen die Probe vor dem Inkontaktbringen mit der biotinylierten Komponente und mit der Biotin-bindenden Komponente mit einer Biotin-bindenden Substanz in Kontakt gebracht wird.

Der vorliegenden Erfindung liegt somit die Aufgabe zugrunde, alternative Verfahren und Mittel bereitzustellen, welche es erlauben, effizient eine etwaige Biotininterferenz in Bindungstesten zu neutralisieren.

Diese Aufgabe wird durch die Gegenstände der unabhängigen Ansprüche gelöst. In den abhängigen Ansprüchen sind weitere vorteilhafte Aspekte der Erfindung reflektiert.

Im Wesentlichen wird die Aufgabe dadurch gelöst, dass in einem Bindungstest zum Nachweis eines Analyten in einer Probe die Probe vor dem oder während des Inkontaktbringen(s) mit einer biotinylierten Komponente und mit einer Biotin-bindenden Komponente mit einer adsorbierenden Substanz in Kontakt gebracht wird, wobei die adsorbierende Substanz eine poröse Struktur mit einer Gesamtoberfläche (S_{Bet}) von 300 bis 6500 m² g⁻¹ hat und mindestens Makro-, Meso- und Mikroporen aufweist, wobei das Volumen der Mikroporen (V_{mic}) 0,15 bis 0,75 cm³ g⁻¹ beträgt.

Dies hat den Effekt, das etwaiges in einer Probe enthaltenes Biotin effizient und sehr zeitsparend gebunden und damit seine das Testergebnis störenden Wirkungen neutralisiert werden. Die auf einer Biotin-Avidin/Streptavidin-Interaktion beruhenden Testschritte können dann ohne Beeinflussung durch freies Biotin durchgeführt werden. Damit sind, je nach TestFormat, falsch niedrige oder falsch hohe Ergebnisse nahezu ausgeschlossen. Die Sicherheit der Testdurchführung wird damit signifikant gesteigert. Weiterhin ist eine bisher üblicherweise durchgeführte Biotin-Titerbestimmung zur Abschätzung des Risikos nicht mehr notwendig.

Gegenstand der vorliegenden Erfindung ist also ein Verfahren zur Neutralisation einer Biotininterferenz in einem Bindungstest zum Nachweis eines Analyten in einer Probe, wobei in dem Bindungstest die Probe mindestens mit einer biotinylierten Komponente und mit einer Biotin-bindenden Komponente in Kontakt gebracht wird und wobei die Probe vor dem oder während des Inkontaktbringen(s) mit der biotinylierten Komponente und mit der Biotin-bindenden Komponente mit einer adsorbierenden Substanz in Kontakt gebracht wird, wobei die adsorbierende Substanz eine poröse Struktur mit einer Gesamtoberfläche (S_{Bet}) von 300 bis 6500 m² g⁻¹ hat und mindestens Makro-, Meso- und Mikroporen aufweist, wobei das Volumen der Mikroporen (V_{mic}) 0,15 bis 0,75 cm³ g⁻¹ beträgt.

Gemäß der vorliegenden Erfindung hat die adsorbierende Substanz eine poröse Struktur. Die Porosität und Porenstruktur ist vorzugsweise durch den Parameter der Gesamtoberfläche (S_{Bet}) charakterisiert, die im Bereich von 300 bis 6500 m² g⁻¹ liegen kann. In speziellen Ausführungsformen kann die Gesamtoberfläche 300 bis 600 m² g⁻¹ betragen oder 400 bis 3500 m² g⁻¹ oder 400 bis 5000 m² g⁻¹. In weiteren speziellen Ausführungsformen kann die Gesamtoberfläche 300, 400, 500, 600, 700, 800, 900, 1000, 1500, 2000, 2500, 2600, 2700, 2800, 2900, 3000, 3100, 3200, 3300, 3400, 3500, 4000, 4500, 5000, 5500, 6000 oder 6500 m² g⁻¹ oder jeden Wert zwischen den genannten Werten betragen. Bevorzugt ist eine Gesamtoberfläche von 2000 bis 3500 m² g⁻¹.

Darüber hinaus weist die Substanz mindestens Makro-, Meso- und Mikroporen auf. Eine "Makropore" ist eine Pore, die einen Durchmesser von mehr als 50 nm hat. Eine "Mesopore" ist eine Pore, die einen Durchmesser von 2 bis 50 nm hat. Eine "Mikropore" ist eine Pore, die einen Durchmesser von weniger als 2 nm hat. Typischerweise erfolgt die Adsorption von Biotin im Bereich der Mikroporen, weshalb die Volumina der Mikroporen ein Indikator für die Adsorptionsfähigkeit der Substanz sind.

Das Volumen der Mikroporen (V_{mic}) der erfindungsgemäßen adsorbierenden Substanz beträgt darüber hinaus 0,15 bis 0,75 cm³ g⁻¹. In speziellen Ausführungsformen kann das Volumen der Mikroporen 0,15 bis 0,25, 0,2 bis 0,5, 0,3 bis 0,75, 0,15 bis 0,2 oder 0,25 bis 0,65 cm³ g⁻¹ betragen. Das Volumen der Mikroporen kann weiterhin einen Wert von 0,15, 0,2, 0,25, 0,3, 0,35, 0,4, 0,45, 0,5, 0,55, 0,6, 0,65, 0,7 oder 0,75 cm³ g⁻ oder jeden dazwischenliegenden Wert aufweisen.

Eine erfindungsgemäße adsorbierende Substanz zeichnet sich dadurch aus, dass sie Biotin an ihrer Oberfläche, d.h. an der Grenzfläche zwischen der Festphase und der flüssigen Phase anreichert. Die Adsorption kann abhängig von der verwendeten Substanz eine physikalische oder eine chemische Adsorption sein. Eine "physikalische Adsorption" ist typischerweise ein physikalischer Prozess, bei dem Moleküle auf der Oberfläche eines anderen Stoffes haften bleiben und sich auf dessen Oberfläche anreichern. Die Kräfte, die die Anhaftung verursachen, sind Van-der-Waals-Kräfte. Typischerweise gelangen die Moleküle durch ihre ungerichtete, thermisch getriebene Molekularbewegung zur Grenzfläche. Bei einer "chemischen Adsorption" werden die Moleküle durch chemische Bindungen auf die Oberfläche eines Feststoffes gebunden und verändern dabei auch den Feststoff, d.h. gehen mit diesem bspw. kovalente Bindungen ein.

Eine bevorzugte adsorbierende Substanz ist Aktivkohle, eine Metall-organische Gerüstverbindung (MOF), Zeolith, Ton, ein poröses organisches Polymer (POP), Hydrotalkit, ein organisch-anorganisches Hybrid, ein poröses Metalloxid, ein Lithium-Zirkonat oder ein mesoporöses Silica-Material (MSM).

Aktivkohle besteht überwiegend aus Kohlenstoff mit hochporöser Struktur. Die Poren sind typischerweise offenporig und untereinander verbunden. Die Gesamtoberfläche beträgt im Regelfall zwischen 300 und 3000 m² g⁻¹, bspw. 2900 oder 3000 m² g⁻¹. Die Dichte von Aktivkohle liegt typischerweise im Bereich von 0,2 bis 0,6 g/cm³. Aktivkohle weist neben Mikroporen, Mesoporen und Makroporen noch Submikroporen einer Größe von < 0,4 nm auf. Die Makro- und Mesoporen sind typischerweise die Zugangswege für Gase oder Flüssigkeiten in das Innere der Aktivkohle und bedingen die Diffusions- und Stofftransportvorgängen in innere Bereiche der Substanz. Die Adsorption erfolgt an der Oberfläche der Mikroporen.

Aktivkohle kann in allen dem Fachmann bekannten nützlichen Formen und Varianten verwendet werden. Beispielsweise kann Aktivkohle in Tablettenform eingesetzt werden. In besonders bevorzugten Ausführungsformen werden DOAC-Stop oder DOAC-Remove Aktivkohle-Tabletten verwendet. DOAC-Stop Tabletten, die beispielsweise von der Firma Haematex vertrieben werden, bestehen aus spezifischen Aktivkohlemischungen. DOAC-Remove Aktivkohle Tabletten, die von der Firma Coachrom oder 5-Diagnostics AG vertrieben werden, bestehen ebenfalls aus spezifischen Aktivkohlemischungen. Weitere Aktivkohleformen, die im Rahmen der vorliegenden Erfindung verwendet werden können, umfassen Produkte mit der CAS-Nr. 7440-44-0. Weitere Details, auch zu weiteren, ebenfalls in der Erfindung vorgesehenen Aktivkohleformen, sind dem Fachmann bekannt oder können aus geeigneten Literaturquellen, wie bspw. Barnes et al., 2009, Carbon, 47, 1887-1895, entnommen werden.

"Metall-organische Gerüstverbindungen (MOFs)" sind mikroporöse Materialien, die aus anorganischen Baueinheiten, den "Inorganic building units", und organischen Molekülen als Verbindungselementen zwischen den anorganischen Baueinheiten aufgebaut sind. Metall-organische Gerüste sind typischerweise kristallin und lassen sich als Koordinationspolymere oder Koordinationsnetzwerke mit einem offenen Gerüst charakterisieren, welches Poren enthält. Beispiele von MOFs, wie sie im Rahmen der vorliegenden Erfindung eingesetzt werden können, sind MOF-5, das aus Zn₄O-Tetraedern besteht, an die organische Liganden in Form von 1,4-Benzoldicarboxyl(BDC) angelagert sind, MOF-177 (mit BTB), MOF-180 (mit BTE), MOF-200 (mit BBC), MOF-205 (mit BTB und NDC), MOF-210 (mit BTE und BPDC) oder CD-MOFs, die als Linker Cyclodextrine und als Konnektoren Alkalimetallkationen verwenden. Weitere Details sind dem Fachmann bekannt oder können aus geeigneten Literaturquellen, wie bspw. Reddy et al., 2021, RSV Adv., 11, 12658, entnommen werden.

Der Begriff "Zeolith" bezieht sich auf kristalline Alumosilikate. Die Zusammensetzung der Stoffgruppe Zeolithe wird typischerweise mit Mⁿ⁺_{x/n} [(AlO₂)⁻ₓ (SiO₂)_{y}] · z H₂O angegeben, wobei n die Ladung des Kations M bezeichnet und 1 oder 2 sein kann. M ist typischerweise ein Kation eines Alkali- oder Erdalkalimetalls. Diese Kationen werden zum elektrischen Ladungsausgleich der negativ geladenen Aluminium-Tetraeder benötigt, sind nicht in das Haupt-Gitter des Kristalls eingebaut, sondern halten sich in Hohlräumen des Gitters auf und lassen sich im Gitter bewegen. z gibt an, wieviele Wassermoleküle vom Kristall aufgenommen wurden. Das molare Verhältnis von SiO₂ zu AlO₂ bzw. y/x in der Summenformel wird als Modul bezeichnet. Zeolithe bestehen somit aus einer mikroporösen Gerüststruktur aus AlO₄⁻- und SiO₄-Tetraedern, wobei die Aluminium- und Silizium-Atome untereinander durch Sauerstoffatome verbunden sind. Je nach Strukturtyp ergibt sich dadurch eine Struktur aus gleichförmigen Poren und/oder Kanälen, in denen Stoffe adsorbiert werden können. Beispiele von Zeolithen, wie sie im Rahmen der vorliegenden Erfindung eingesetzt werden können, umfassen MFI Zeolithe, CHA Zeolithe, LTA Zeolithe, Zeolith A, Zeolith X, Zeolith L, ZSM 11, ZSM 5, oder Zeolith Y. Zeolithe weisen im Regelfall eine Porengröße im Mikroporenbereich von ca. 2 nm auf. Weitere Details sind dem Fachmann bekannt oder können aus geeigneten Literaturquellen, wie bspw. Reddy et al., 2021, RSV Adv., 11, 12658, entnommen werden.

Der Begriff "Ton" bezeichnet im Kontext der vorliegenden Erfindung Schichtsilikate, die eine schichtartige Kristallstruktur aus Silizium und Sauerstoff sowie Wasserstoff und meist Magnesium und Aluminium aufweisen. Weitere Details sind dem Fachmann bekannt oder können aus geeigneten Literaturquellen, wie bspw. Reddy et al., 2021, RSV Adv., 11, 12658, entnommen werden.

"Poröse organische Polymere (POPs)" sind eine Klasse mehrdimensionaler poröser Netzwerkmaterialien, die über starke kovalente Bindungen zwischen verschiedenen organischen Bausteinen mit unterschiedlichen Geometrien und Topologien aufgebaut sind. POPs können im Allgemeinen basierend auf ihrem Grad an Fernordnung in zwei Kategorien eingeteilt werden, amorphe und kristalline POPs. Amorphe POPs umfassen hauptsächlich hypervernetzte Polymere (HCPs), Polymere mit intrinsischer Mikroporosität (PIMs), konjugierte mikroporöse Polymere (CMPs) und poröse aromatische Gerüste (PAFs), während kristalline POPs kovalente organische Gerüste (COFs) umfassen. Sie haben ein geringes Gewicht, eine gut entwickelte inhärente Porosität, große Stabilität, sind vorkonfigurierbar und weisen einstellbare Strukturen und Funktionen auf. Vorteilhafterweise kann ihre poröse Struktur, Porengröße, spezifischen Oberflächenbereiche und Funktionen entworfen und durch Einführen spezifischer funktionaler Bausteine beeinflusst werden. Weitere Details sind dem Fachmann bekannt oder können aus geeigneten Literaturquellen, wie bspw. Reddy et al., 2021, RSV Adv., 11, 12658, entnommen werden.

"Hydrotalkit" ist ein Mineral aus der Klasse der Carbonate. Es kristallisiert im trigonalen Kristallsystem mit der chemischen Zusammensetzung Mg₆Al₂[(OH) ₁₆|CO₃] ·4H₂O und entwickelt meist durchsichtige, tafelige Kristalle bis etwa 4 mm Größe mit seiden- bis wachsglänzenden Kristallflächen. Weitere Details sind dem Fachmann bekannt oder können aus geeigneten Literaturquellen, wie bspw. Reddy et al., 2021, RSV Adv., 11, 12658, entnommen werden.

Ein "organisch-anorganisches Hybrid" ist eine Materialklasse, deren Struktur sowohl organische als auch anorganische Einheiten umfasst, die auf molekularer Ebene miteinander wechselwirken. Diese Materialien werden auf der Grundlage der Wechselwirkung zwischen organischen und anorganischen Komponenten in zwei Klassen eingeteilt. In Klasse I sind organische und anorganische Komponenten eingebettet, und es gibt schwache Wechselwirkungen wie Wasserstoffbrücken, Van-der-Waals-, π-π- oder schwache elektrostatische Wechselwirkungen zwischen ihnen. In Klasse II sind diese beiden Komponenten durch starke kovalente oder koordinative Bindungen miteinander verbunden. Organisch-anorganische Hybridpolymere können beispielsweise erhalten werden durch Sol-Gel-Verfahren, Selbstaufbauprozesse oder Assemblieren oder Dispergieren von Nanobausteinen. Beispiele umfassen Kern-Schale-Nanokomposite (CSNs), polystyrolbeschichtete Fe₂O₃-Partikel oder interpenetrierende Netzwerke (IPNs). Weitere Details sind dem Fachmann bekannt oder können aus geeigneten Literaturquellen, wie bspw. Reddy et al., 2021, RSV Adv., 11, 12658, entnommen werden.

Ein "poröses Metalloxid (MO)" ist eine Verbindung zwischen einem Metall und Sauerstoff. Durch strukturdirigierende Maßnahmen auf Basis von Kohlenstoff oder Silikamatrizen mesoporöser Form gibt es inzwischen poröse Metalloxide verschiedener Ausprägung, wie bspw. TiO₂, ZrO₂, Al₂O₃, V₂O₅, MoO₃, Fe₂O₃, MgAl₂PO₄, MgO₂, CeO₂ oder MnO₂. Weitere Details sind dem Fachmann bekannt oder können aus geeigneten Literaturquellen, wie bspw. Reddy et al., 2021, RSV Adv., 11, 12658, entnommen werden.

Lithium-Zirkonat kann ebenfalls poröse Strukturen ausbilden. Weitere Details sind dem Fachmann bekannt oder können aus geeigneten Literaturquellen, wie bspw. Reddy et al., 2021, RSV Adv., 11, 12658, entnommen werden.

"Mesoporöses Silica-Material (MSM)" zeichnen sich durch eine hohe Symmetrie der Porenanordnung, enge Porenweitenverteilungen und Oberflächen von bis zu 1000 m²/g aus. Die Porenwände der MSM bestehen typischerweise aus reinem, amorphem SiO₂. Beispiele von MSM umfassen die M41S-Familie, bspw. MCM41, das hexagonal angeordnete, zylindrische Poren aufweist. Ein weiteres Beispiel ist die Familie SBA, deren Mitglieder eine mit MCM41 vergleichbare, hexagonale Porenstruktur aufweisen und sich auf Grund ihrer hohen Porenwandstärke durch eine hohe hydrothermale Stabilität auszeichnen und außerdem auf Grund der PO-Seitenketten der polymeren Templates über eine kombinierte Mikro- und Mesoporosität verfügen, bspw. SBA15. Weitere Klassen umfassen KIT-6 und MCM-48. Weitere Details sind dem Fachmann bekannt oder können aus geeigneten Literaturquellen, wie bspw. Reddy et al., 2021, RSV Adv., 11, 12658, entnommen werden.

Die Probe kann mit der adsorbierenden Substanz in Kontakt gebracht werden, indem die Probe mit der Substanz in Form eines Pulvers, Granulats oder Mikrofasergemisches zu einer Suspension vermischt wird. Dadurch wird das Biotin aus der Probe an die adsorbierende Substanz angelagert und somit quantitativ der Probe entzogen, welche ohne weitere Interferenz von Biotin in einem Bindungstest ausgewertet werden kann. Die Vermischung der Probe mit der adsorbierenden Substanz wird vor der Durchführung von Nachweisschritten für einen Analyten durchgeführt.

Die Probe kann dann vor dem anschließenden Inkontaktbringen mit der biotinylierten Komponente und mit der Biotin-bindenden Komponente aus der Suspension von der adsorbierenden Substanz abgetrennt werden. Die Abtrennung kann beispielsweise über Zentrifugation oder Filtrierung erfolgen.

Alternativ kann die Probe mit der adsorbierenden Substanz in Kontakt gebracht werden, indem die Probe mit einer mit der Substanz beschichteten Festphase in Kontakt gebracht wird.

Die adsorbierende Substanz wie oben beschrieben kann in spezifischen Ausführungsformen auf einer Festphase aufgebracht sein. Der Begriff "Festphase" im Sinne der Erfindung bezieht sich auf einen Gegenstand, der aus porösem und/oder nicht porösem, wasserunlöslichem Material besteht und verschiedene Formen aufweisen kann wie z.B. ein Gefäß, eine Pipette, ein Röhrchen, eine Mikrotitrationsplatte, eine Kugel, ein Mikropartikel, Stäbchen, ein Streifen, Filter- oder Chromatographiepapier etc. In der Regel ist die Oberfläche der Festphase hydrophil. Die Festphase kann aus verschiedenen Materialien bestehen wie z.B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere, wie z.B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik; Silikat; Glas; Metalle, z.B. magnetisierbare Metalle wie Eisen, oder Edelmetalle wie Gold und Silber; Magnetit; Mischungen oder Kombinationen derselben; etc.

Die Festphase kann in bevorzugten Ausführungsformen ein Filter (wie z.B. in der WO 98/09167 A1 beschrieben), die Oberfläche eines Flüssigkeitsbehälters, eine Pipettenspitze, oder ein Partikel aus Latex, Sepharose, Agarose, Kunststoff, Glas, Protein, Alginat, Silikat oder Metall sein.

In besonderen Ausführungsformen ist die Festphase ein magnetisierbarer Partikel, der mittels Anlegen eines Magnetfeldes aus einer Mischung oder Suspension entnommen werden oder an einer bestimmten Position immobilisiert werden kann.

Die Beschichtung einer Festphase mit einer adsorbierenden Substanz wird so durchgeführt, dass die Porosität der Substanz erhalten bleibt und damit eine Anlagerung von Biotin möglich wird. Je nach Substanzklasse müssen unterschiedliche Beschichtungsdicken und -formen berücksichtigt werden. Derartige Details sind dem Fachmann bekannt und können aus geeigneten Literaturquellen, wie bspw. Kim et al., 2016, Scientific Reports, 6, 21182 oder Rocher et al., 2008, Water Res, 42(4-5), 1290-8, entnommen werden. Zur Reduzierung der Menge an Biotin wird die beschriebene Festphase mit der Probe in Kontakt gebracht. Dabei kann Biotin über die vorhandenen Poren und entsprechende Wechselwirkungen an die Festphase angelagert werden. Damit ist es quantitativ der Probe entzogen, welche ohne weitere Interferenz von Biotin in einem Bindungstest ausgewertet werden kann.

In einer weiteren bevorzugten Ausführungsform des Verfahrens ist die adsorbierende Substanz mit einem oder mehreren Blockierungsproteinen oder Blockierungspeptiden vorbehandelt, vorzugsweise mit einem oder mehreren Blockierungsproteinen aus der Gruppe Dextran, Albumin, Polygeline und Milcheiweiß.

Alternativ oder zusätzlich kann die adsorbierende Substanz mit Oberflächenblockierern beschichtet sein. Ein "Oberflächenblockierer" wird im Kontext der vorliegenden Erfindung als Molekül verstanden, das freie Bindungsstellen an Oberflächen von adsorbierenden Substanzen absättigt und so dazu beiträgt, dass Analyte und Testkomponenten nicht an Oberflächenstrukturen der Substanz abgefangen werden. Die Verfälschung eines Testergebnisses wird dadurch verhindert. Beispiele von Oberflächenblockierern, die im Rahmen der Erfindung eingesetzt werden können, umfassen Albumin, bspw. BSA oder HSA, Dextrane, d.h. hochmolekulare verzweigte Polysaccharide, bspw. Aminodextran, Parylen, Polyxylen, Sepharose, d.h. eine bearbeitete Form des Polymers Agarose, oder Polygelin, d.h. ein Polymer aus Harnstoff und hydrolysierter Gelatine, bspw. Haemaccel. Weitere Oberflächenblockierer sind Casein bzw. Milcheiweiß oder auf Casein basierende Reagenzien, bspw. die kommerziell erhältliche Blocking Solution von Candor, die auf hochreinem Casein basiert, das durch chemische Modifikationen verändert wurde. Dabei entsteht ein Spektrum unterschiedlich großer Fragmente des Caseins. Weitere Möglichkeiten stellen Peptidkombinationen dar, die chemisch modifiziert wurden und so in kleine Lücken an der Oberfläche eindringen können, bspw. das Produkt SmartBlock. In weiteren Ausführungsformen kann nach Beschichtung mit einem Oberflächenblockierer eine zusätzliche Stabilisierung mit Zuckerlösungen oder kommerziellen Lösungen wie Liquid Plate Sealer erfolgen. Diese Stabilisierer legen sich um die Oberflächenblockierer und verhindern deren Degradierung und Entfernung.

Es ist weiterhin bevorzugt, dass die adsorbierende Substanz in Form von Polymer-beschichteten Substanz-Kügelchen mit einer Porengröße mit Durchlässigkeit für Biomoleküle mit einer Masse < 40 kDa bereitgestellt wird.

Alternativ kann die adsorbierende Substanz in Form von Polymer-beschichteten Kügelchen oder Partikeln bereitgestellt werden. So können beispielsweise Partikel auf Basis von Acrylsäure, Methacrylsäure, Methylmethacrylsäure, P-Vinylbenzoesäure, 2-Ethenybutandiolsäure, 1-Ethyl-4-Vinylbenzol, Styren, 4-Vinylpyridin, 1-Vinylimidazol, Acrylamid, Methacrylamid, Alginat oder Silikat erzeugt werden. Die Partikel können weiterhin mit magnetisierbaren Komponenten versehen sein, z.B. Eisenoxid. Es ist bevorzugt, dass die Partikel eine Porengröße aufweisen, die eine Durchlässigkeit für Biomoleküle mit einer Masse von < 40 kDa, z.B. 35 kDa, 30 kDa, 25 kDa, 20 kDa, 15 kDa, 10 kDa, 5 kDa, 2 kDa, 1 kDa, 0,5 kDa, 0,25 kDa oder kleiner besitzt. Die Porosität der Partikel kann über die Art der Substanz gesteuert werden. Weiterhin können Matrizenstrukturen zur Generierung von Poren verwendet werden, insbesondere bei Verwendung von Metalloxiden. Derartige Details sind dem Fachmann bekannt und können aus geeigneten Literaturquellen, wie bspw. Kim et al., 2016, Scientific Reports, 6, 21182 oder Rocher et al., 2008, Water Res, 42(4-5), 1290-8, entnommen werden.

Der Begriff "Biotininterferenz", wie hierin verwendet, bezieht sich auf die Anwesenheit von Biotin in einem Bindungstest zum Nachweis eines Analyten in einer Probe, wobei das Biotin nicht als funktionelle Komponente des Bindungstests vorliegt, sondern aus der zu untersuchenden Probe stammt. Dabei interferiert dieses freie Biotin mit der dem Bindungstest zugrundeliegenden Interaktion einer biotinylierten Testkomponente mit einem Biotin-spezifischen Bindungspartner, z.B. Streptavidin oder strukturell oder funktionelle ähnlichen Komponenten, und beeinflusst bzw. verfälscht das Ergebnis des Bindungstests, da es an den Biotin-spezifischen Bindungspartner bindet und diesen dadurch für Nachweisreaktionen blockiert.

Der Begriff "Bindungstest" umfasst im Wesentlichen Verfahren für die Detektion von Analyten, bei denen eine Analytenthaltende Probe mit einem oder mehreren Analyt-spezifischen Bindungspartnern in Kontakt gebracht wird und der oder die entstehenden Komplex(e) aus Analyt und Analyt-spezifischen Bindungspartner(n) quantitativ bestimmt werden. Ein klassisches Bindungstestformat ist z.B. der sogenannte Sandwich-Assay, bei dem ein Analyt von einem Fänger- und einem Detektions-Bindungspartner gebunden wird. Außerdem gibt es weitere zahlreiche dem Fachmann hinreichend bekannte Bindungstestformate (kompetitive Bindungsteste, heterogene Bindungsteste, homogene Bindungsteste etc.). Als Analyt-spezifische Bindungspartner kommen in den Bindungstesten vorzugweise Antikörper, Antikörperfragmente, Affimere oder Aptamere zum Einsatz.

In den durch die Erfindung verbesserten Bindungstesten wird die Probe mit mindestens einer biotinylierten Komponente und mit einer Biotin-bindenden Komponente in Kontakt gebracht. Je nach Testformat sind die biotinylierte Komponente und die Biotin-bindende Komponente so aufeinander abgestimmt, dass die Interaktion von Biotin und Biotin-Bindungspartner eine Anreicherung bzw. Abtrennung des Analyten oder eine Signalverstärkung oder eine andere für das Testformat zweckmäßige Funktion erfüllt. Dazu können die Biotin-bindende Komponente und/oder die biotinylierte Komponente ein Peptid, ein Protein, einen Antikörper, ein Antikörperfragment, oder ein Affimer oder Aptamer enthalten.

Unter einer "biotinylierten Komponente" ist also eine Testkomponente zu verstehen, die eine funktionelle Einheit, beispielsweise (i) einen Bindungspartner aus der Gruppe Peptid, Protein, Antikörper, Antikörperfragment, Affimer und Aptamer, (ii) eine Komponente eines signalbildenden Systems und/oder (iii) eine Festphase (z.B. Partikel) umfasst, an die ein oder mehrere Biotinmoleküle direkt oder indirekt gekoppelt sind.

Unter einer "Biotin-bindenden Komponente" ist analog eine Testkomponente zu verstehen, die über Bindungsstellen für die spezifische Bindung von Biotin verfügt. Vorzugsweise handelt es sich bei der "Biotin-bindenden Komponente" um eine funktionelle Einheit, beispielsweise (i) einen Bindungspartner aus der Gruppe Peptid, Protein, Antikörper, Antikörperfragment, Affimer und Aptamer, (ii) eine Komponente eines signalbildenden Systems und/oder (iii) eine Festphase (z.B. Partikel), an die ein oder mehrere Biotin-bindende Moleküle direkt oder indirekt gekoppelt sind. Bevorzugte Biotin-bindende Moleküle sind Avidin, Streptavidin, Tamavidin 1 oder 2, Shwanavidin, und/oder Rhizavidin, Bradavidin, Burkavidin, Zebavidin, Xenavidin und/oder Strongavidin.

Es ist weiter bevorzugt, dass die Biotin-bindende Komponente und/oder die biotinylierte Komponente ein Peptid, ein Protein, einen Antikörper, ein Antikörperfragment, ein Affimer oder ein Aptamer enthält.

Die Anwendbarkeit der beschriebenen Komponenten ist nicht auf die oben im Detail ausgeführten Standardszenarien beschränkt, sondern kann je nach Bedarf, Analyt und gewünschtem Messergebnis variiert und angepasst werden.

In einer zusätzlichen, bevorzugten Ausführungsform enthält die biotinylierte Komponente eine erste Komponente eines signalbildenden Systems und die Biotin-bindende Komponente eine zweite Komponente des signalbildenden Systems, wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

In einer weiteren bevorzugten Ausführungsform kann die erste Komponente des signalbildenden Systems ein chemilumineszierendes Agens sein und die zweite Komponente des signalbildenden Systems ein Photosensitizer, oder umgekehrt.

Ein "Photosensitizer" ist ein Stoff, dessen Absorptionsbereich im Wellenlängenbereich des eingestrahlten Lichtes liegt und als photochemischer Umsetzer wirkt, d.h. er überträgt die Lichtenergie auf ein zweites Molekül, das andere Absorptionseigenschaften hat, aber nach der Übertragung der Lichtenergie reagieren kann. Beispiele sind etwa Phthalocyanin. Weiterhin kann ein System wie AlphaLISA verwendet werden, das Lumineszenz-Sauerstoff-Channeling-Chemie zur Detektion einsetzt. Weitere Details können bspw. Pulido-Olmo et al., 2017, Frontiers in Immunology, 8, Article 853 entnommen werden.

Ein "chemilumineszierendes Agens" ist ein Agens, das nach chemischer Reaktion elektromagnetische Strahlung im Bereich des ultravioletten oder sichtbaren Lichts emittiert. Beispiele für chemilumineszierende Verbindungen sind Luminol oder Imidazol. Im weiteren Sinne sind ebenfalls biolumineszente Proteine umfasst, z.B. Luciferin, Luciferase, grün fluoreszierendes Protein (GFP), mCherry, mOrange, TagBFP, Cerulean, Citrine, mTurquoise, rot fluoreszierendes Protein (RFP), gelb fluoreszierendes Protein (YFP) und Derivate davon. Weitere chemilumineszierende Verbindungen, die im Rahmen der vorliegenden Erfindung verwendet werden können, umfassen 6-FAM, HEX, TET, ROX, Cy2, Cy3, Cy5, Cy7, Texas Red oder Rhodamine, PerCP, Pacific Blue, APC, Alexa 405, 430 , 488, 546, 559, 594, 633, 660, 674, 680, 700, Cascade Blue, TAMRA, Dabcyl, Black Hole Quencher, BHQ-1 oder BHQ-2. Weitere Details, auch bezüglich eines Testformates auf Basis der genannten Elemente, sind dem Fachmann bekannt oder können aus Pulido-Olmo et al., 2017, Frontiers in Immunology, 8, Article 853 entnommen werden.

Die zu analysierende Probe ist vorzugsweise eine biologische Probe. Der Begriff "biologische Probe" bezieht sich allgemein auf eine Tier-Körperflüssigkeit, z.B. eine Wirbeltier-Körperflüssigkeit, vorzugsweise auf eine Säugetier-Körperflüssigkeit, besonders bevorzugt auf eine menschliche Köperflüssigkeit. Umfasst sind alle Arten von Körperflüssigkeiten, in denen Analyten potenziell gefunden und/oder untersucht werden können. Bevorzugt sind insbesondere Vollblut, Blutplasma, Blutserum, Speichel, Liquor oder Tränenflüssigkeit.

In einem weiteren Aspekt bezieht sich die Anmeldung auf die Verwendung einer adsorbierenden Substanz, wobei die Substanz eine poröse Struktur mit einer Gesamtoberfläche (S_{Bet}) von 300 bis 6500 m² g⁻¹ hat und mindestens Makro-, Meso- und Mikroporen aufweist, wobei das Volumen der Mikroporen (V_{mic}) 0,15 bis 0,75 cm³ g⁻¹ beträgt, zur Neutralisation einer Biotininterferenz in einem Bindungstest zum Nachweis eines Analyten in einer Probe, wobei in dem Bindungstest die Probe mindestens mit einer biotinylierten Komponente und mit einer Biotin-bindenden Komponente in Kontakt gebracht wird. Die Substanz ist vorzugweise Aktivkohle, eine Metall-organische Gerüstverbindung (MOF), Zeolith, Ton, ein poröses organisches Polymer (POP), Hydrotalkit, ein organisch-anorganisches Hybrid, ein poröses Metalloxid, ein Lithium-Zirkonat oder ein mesoporöses Silica-Material (MSM). Die Komponenten und Elemente des Bindungstests entsprechen den oben beschriebenen Komponenten und Elementen.

Die Verwendung der adsorbierenden Substanzen zur Neutralisierung einer Biotininterferenz findet idealerweise vor dem Inkontaktbringen von Proben und Testkomponenten statt, z.B. durch eine Vorinkubation/Vorbehandlung der Probe mit der adsorbierenden Substanz. Die erfindungsgemäße Verwendung ist dabei nicht auf spezifische Testformate oder spezifische Bindungstests eingeschränkt, sondern umfasst alle Biotin-basierten Interaktionsschemata, bei denen ein erhöhter Biotinspiegel in der Probe (potenziell) vorhanden ist oder vermutet wird.

Ferner offenbart ist ein beschichtetes Festphasensystem, das für die Neutralisation einer Biotininterferenz in einem Bindungstest zum Nachweis eines Analyten in einer Probe geeignet ist.

Im Stand der Technik sind diverse Festphasensysteme für die Anwendung in der Biotechnologie oder Biomedizin beschrieben. Sponchia, G. et al., J Nanopart Res (2014) 16:2245 beschreiben mesoporöse Silika-Nanopartikel; DE 10 2010 050 644 A1 beschreibt mit Kohlenstoff geschützte magnetische Nanosphären; Budnyak T.M. et al., J Therm Anal Calorim (2016) 125:1335-1351 beschreiben organomineralische Chitosan-Silika Nanokomposite.

Das hierin beschriebene beschichtete Festphasensystem zeichnet sich dadurch aus, dass
(i) die Beschichtung eine adsorbierenden Substanz umfasst, wobei die Substanz eine poröse Struktur mit einer Gesamtoberfläche (S_{Bet}) von 300 bis 6500 m² g⁻¹ hat und mindestens Makro-, Meso- und Mikroporen aufweist, wobei das Volumen der Mikroporen (V_{mic}) 0,15 bis 0,75 cm³ g⁻¹ beträgt, wobei die Substanz vorzugsweise Aktivkohle, eine Metall-organische Gerüstverbindung (MOF), Zeolith, Ton, ein poröses organisches Polymer (POP), Hydrotalkit, ein organisch-anorganisches Hybrid, ein poröses Metalloxid, ein Lithium-Zirkonat oder ein mesoporöses Silica-Material (MSM) ist; und
(ii) die Festphase ausgewählt ist aus der Gruppe Filter, Oberfläche eines Flüssigkeitsbehälters, Pipettenspitze, und Partikel aus Latex, Sepharose, Agarose, Kunststoff, Glas, Protein, Alginat, Silikat oder Metall.

Die Beschichtung kann wie weiter oben ausgeführt angebracht sein. Typischerweise ist die Beschichtung so angebracht, dass die poröse Struktur der adsorbierenden Substanz nicht beeinträchtigt wird und die funktionalen Eigenschaften bezüglich der Anlagerung von Biotin erhalten bleiben.

Die Festphase kann dabei aus allen geeigneten Materialen ausgewählt werden. Es ist bevorzugt, dass Materialien und Produkte benutzt werden, die einem Kontakt mit der zu analysierenden Probe vor oder ggf. während der Durchführung eines Bindungstests oder Assays ohnehin ausgesetzt sind. Beispielsweise kann dies ein Filter, die Oberfläche eines Flüssigkeitsbehälters oder eine Pipettenspitze sein. Weitere Materialien umfassen Partikel aus Latex, Sepharose, Agarose, Kunststoff, Glas, Protein, Alginat, Silikat oder Metall. Weitere Details sind dem Fachmann bekannt und können aus geeigneten Literaturquellen, wie bspw. Kim et al., 2016, Scientific Reports, 6, 21182 oder Rocher et al., 2008, Water Res, 42(4-5), 1290-8, entnommen werden.

Obwohl die vorliegende Erfindung in Bezug auf bestimmte Ausführungsformen beschrieben wird, ist diese Beschreibung nicht in einem einschränkenden Sinne aufzufassen.

Wie in dieser Beschreibung und in den beigefügten Ansprüchen verwendet, schließen die Singularformen von "ein" und "eine" auch die jeweiligen Pluralformen ein, sofern der Kontext nicht eindeutig etwas anderes vorschreibt.

Im Zusammenhang mit der vorliegenden Erfindung bezeichnen die Begriffe "ungefähr" und "etwa" ein Genauigkeitsintervall, das ein Fachmann verstehen wird, um die technische Wirkung des betreffenden Merkmals noch sicherzustellen. Der Begriff gibt typischerweise eine Abweichung von dem angegebenen Zahlenwert von ±20 %, vorzugsweise ±15 %, stärker bevorzugt ±10 % und noch stärker bevorzugt ±5 % an.

Es versteht sich, dass der Begriff "umfassend" nicht einschränkend ist.

Wenn im Folgenden eine Gruppe so definiert wird, dass sie mindestens eine bestimmte Anzahl von Ausführungsformen umfasst, soll dies auch eine Gruppe umfassen, die vorzugsweise nur aus diesen Ausführungsformen besteht.

Darüber hinaus werden die Begriffe "(i)", "(ii)", "(iii)" oder "(a)", "(b)", "(c)", "(d)" oder "erste", "zweite", "dritte" usw. und dergleichen in der Beschreibung oder in den Ansprüchen zur Unterscheidung zwischen ähnlichen Elementen und nicht notwendigerweise zur Beschreibung einer sequenziellen oder chronologischen Reihenfolge verwendet.

Es versteht sich, dass die so verwendeten Begriffe unter geeigneten Umständen austauschbar sind und dass die hier beschriebenen Ausführungsformen der Erfindung in anderer Reihenfolge als hier beschrieben verwendet werden können. Falls sich die Begriffe auf Schritte einer Methode, eines Verfahrens oder einer Verwendung beziehen, gibt es keine Zeit- oder Zeitintervallkohärenz zwischen den Schritten, d.h. die Schritte können gleichzeitig ausgeführt werden oder es können Zeitintervalle von Sekunden, Minuten, Stunden, Tagen, Wochen usw. zwischen solchen Schritten vorliegen, sofern nichts anders angegeben ist.

Es versteht sich, dass diese Erfindung nicht auf die hier beschriebenen speziellen Verfahren, Protokolle usw. beschränkt ist, da diese variieren können. Es versteht sich auch, dass die hierin verwendete Terminologie nur dem Zweck dient, bestimmte Ausführungsformen zu beschreiben, und nicht dazu gedacht ist, den Umfang der vorliegenden Erfindung einzuschränken, der nur durch die beigefügten Ansprüche begrenzt wird.

Sofern nicht anders definiert, haben alle hierin verwendeten technischen und wissenschaftlichen Begriffe die gleichen Bedeutungen, wie sie üblicherweise von einem Durchschnittsfachmann verstanden werden.

Die folgenden Beispiele und Figuren und dienen der Veranschaulichung. Es versteht sich daher, dass die Beispiele und Figuren nicht als einschränkend ausgelegt werden sollen. Der Fachmann kann sich weitere Modifikationen der hier dargelegten Prinzipien klar vorstellen.

## Patentansprüche

1. Verfahren zur Neutralisation einer Biotininterferenz in einem Bindungstest zum Nachweis eines Analyten in einer Probe, wobei in dem Bindungstest die Probe mindestens mit einer biotinylierten Komponente und mit einer Biotin-bindenden Komponente in Kontakt gebracht wird, **dadurch gekennzeichnet, dass** die Probe vor dem oder während des Inkontaktbringen(s) mit der biotinylierten Komponente und mit der Biotin-bindenden Komponente mit einer adsorbierenden Substanz in Kontakt gebracht wird, wobei die adsorbierende Substanz eine poröse Struktur mit einer Gesamtoberfläche (S_{Bet}) von 300 bis 6500 m² g⁻¹ hat und mindestens Makro-, Meso- und Mikroporen aufweist, wobei das Volumen der Mikroporen (V_{mic}) 0,15 bis 0,75 cm³ g⁻¹ beträgt.

2. Verfahren gemäß Anspruch 1, wobei die adsorbierende Substanz Aktivkohle, eine Metall-organische Gerüstverbindung (MOF), Zeolith, Ton, ein poröses organisches Polymer (POP), Hydrotalkit, ein organisch-anorganisches Hybrid, ein poröses Metalloxid, ein Lithium-Zirkonat oder ein mesoporöses Silica-Material (MSM) ist.

3. Verfahren gemäß einem der vorhergehenden Ansprüche, wobei die Probe mit der adsorbierenden Substanz in Kontakt gebracht wird, indem die Probe mit der Substanz in Form eines Pulvers, Granulats oder Mikrofasergemisches zu einer Suspension vermischt wird.

4. Verfahren gemäß Anspruch 3, wobei die Probe vor dem anschließenden Inkontaktbringen mit der biotinylierten Komponente und mit der Biotin-bindenden Komponente aus der Suspension von der adsorbierenden Substanz abgetrennt wird.

5. Verfahren gemäß einem der Ansprüche 1 und 2, wobei die Probe mit der adsorbierenden Substanz in Kontakt gebracht wird, indem die Probe mit einer mit der Substanz beschichteten Festphase in Kontakt gebracht wird.

6. Verfahren gemäß Anspruch 5, wobei die mit der adsorbierenden Substanz beschichtete Festphase ausgewählt ist aus der Gruppe Filter, Oberfläche eines Flüssigkeitsbehälters, Pipettenspitze und Partikel aus Latex, Sepharose, Agarose, Kunststoff, Glas, Protein, Alginat, Silikat oder Metall.

7. Verfahren gemäß einem der vorhergehenden Ansprüche, wobei die adsorbierende Substanz mit einem oder mehreren Blockierungsproteinen oder Blockierungspeptiden vorbehandelt ist, vorzugsweise mit einem oder mehreren Blockierungsproteinen aus der Gruppe Albumin, Polygeline und Milcheiweiß, oder mit Dextran.

8. Verfahren gemäß einem der vorhergehenden Ansprüche, wobei die adsorbierende Substanz in Form von Polymer-beschichteten Substanz-Kügelchen mit einer Porengröße mit Durchlässigkeit für Biomoleküle mit einer Masse < 40 kDa bereitgestellt wird.

9. Verfahren gemäß einem der vorhergehenden Ansprüche, wobei die Biotin-bindende Komponente Biotin-bindendes Avidin, Streptavidin, Tamavidin 1 oder 2, Shwanavidin, Rhizavidin, Bradavidin, Burkavidin, Zebavidin, Xenavidin und/oder Strongavidin enthält.

10. Verfahren gemäß einem der vorhergehenden Ansprüche, wobei die Biotin-bindende Komponente und/oder die biotinylierte Komponente ein Peptid, ein Protein, einen Antikörper, ein Antikörperfragment, ein Affimer oder ein Aptamer enthält.

11. Verfahren gemäß einem der vorhergehenden Ansprüche, wobei die biotinylierte Komponente eine erste Komponente eines signalbildenden Systems und die Biotin-bindende Komponente eine zweite Komponente des signalbildenden Systems enthält und wobei die erste und zweite Komponente des signalbildenden Systems so zusammenwirken, dass ein detektierbares Signal entsteht, wenn die erste und die zweite Komponente des signalbildenden Systems in räumliche Nähe zueinander gebracht werden.

12. Verfahren gemäß Anspruch 11, wobei die erste Komponente des signalbildenden Systems ein chemilumineszierendes Agens ist und die zweite Komponente des signalbildenden Systems ein Photosensitizer ist, oder umgekehrt.

13. Verfahren gemäß einem der vorhergehenden Ansprüche, wobei die Probe eine Körperflüssigkeitsprobe eines Menschen oder eines Tieres ist, vorzugsweise Vollblut, Blutplasma, Blutserum, Urin, Liquor oder Tränenflüssigkeit.

14. Verwendung einer adsorbierenden Substanz, wobei die Substanz eine poröse Struktur mit einer Gesamtoberfläche (S_{Bet}) von 300 bis 6500 m² g⁻¹ hat und mindestens Makro-, Meso- und Mikroporen aufweist, wobei das Volumen der Mikroporen (V_{mic}) 0,15 bis 0,75 cm³ g⁻¹ beträgt, vorzugsweise von Aktivkohle, einer Metallorganischen Gerüstverbindung (MOF), von Zeolith, von Ton, eines porösen organischen Polymers (POP), von Hydrotalkit, eines organisch-anorganischen Hybrids, eines porösen Metalloxids, eines Lithium-Zirkonats oder eines mesoporösen Silica-Materials (MSM), zur Neutralisation einer Biotininterferenz in einem Bindungstest zum Nachweis eines Analyten in einer Probe, wobei in dem Bindungstest die Probe mindestens mit einer biotinylierten Komponente und mit einer Biotin-bindenden Komponente in Kontakt gebracht wird.

## Claims

1. Method for neutralizing biotin interference in a binding assay for detection of an analyte in a sample, wherein the binding assay comprises contacting the sample at least with a biotinylated component and with a biotin-binding component, **characterized in that** the sample is contacted with an adsorbent substance before or during the contacting with the biotinylated component and with the biotin-binding component, wherein the adsorbent substance has a porous structure having a total surface area (S_{Bet}) of from 300 to 6500 m² g⁻¹ and has at least macropores, mesopores and micropores, wherein the volume of the micropores (V_{mic}) is from 0.15 to 0.75 cm³ g⁻¹.

2. Method according to Claim 1, wherein the adsorbent substance is activated carbon, a metal-organic framework (MOF), zeolite, clay, a porous organic polymer (POP), hydrotalcite, an organic-inorganic hybrid, a porous metal oxide, a lithium zirconate or a mesoporous silica material (MSM).

3. Method according to either of the preceding claims, wherein the sample is contacted with the adsorbent substance by mixing the sample with the substance in the form of a powder, granular material or microfibre mixture to form a suspension.

4. Method according to Claim 3, wherein the sample is removed from the suspension of the adsorbent substance before the subsequent contacting with the biotinylated component and with the biotin-binding component.

5. Method according to either of Claims 1 and 2, wherein the sample is contacted with the adsorbent substance by contacting the sample with a solid phase coated with the substance.

6. Method according to Claim 5, wherein the solid phase coated with the adsorbent substance is selected from the group consisting of filter, surface of a liquid container, pipette tip and particle composed of latex, Sepharose, agarose, plastic, glass, protein, alginate, silicate or metal.

7. Method according to any of the preceding claims, wherein the adsorbent substance has been pretreated with one or more blocking proteins or blocking peptides, preferably with one or more blocking proteins from the group consisting of albumin, polygeline and milk protein, or with dextran.

8. Method according to any of the preceding claims, wherein the adsorbent substance is provided in the form of polymer-coated substance beads having a pore size with permeability for biomolecules of a mass < 40 kDa.

9. Method according to any of the preceding claims, wherein the biotin-binding component contains biotin-binding avidin, streptavidin, tamavidin 1 or 2, shwanavidin, rhizavidin, bradavidin, burkavidin, zebavidin, xenavidin and/or strongavidin.

10. Method according to any of the preceding claims, wherein the biotin-binding component and/or the biotinylated component contains a peptide, a protein, an antibody, an antibody fragment, an affimer or an aptamer.

11. Method according to any of the preceding claims, wherein the biotinylated component contains a first component of a signal-forming system and the biotin-binding component contains a second component of the signal-forming system and wherein the first and the second component of the signal-forming system interact in such a way that a detectable signal is formed if the first and the second component of the signal-forming system are brought into physical proximity with one another.

12. Method according to Claim 11, wherein the first component of the signal-forming system is a chemiluminescent agent and the second component of the signal-forming system is a photosensitizer or vice versa.

13. Method according to any of the preceding claims, wherein the sample is a body fluid sample from a human or an animal, preferably whole blood, blood plasma, blood serum, urine, cerebrospinal fluid or lacrimal fluid.

14. Use of an adsorbent substance, wherein the substance has a porous structure having a total surface area (S_{Bet}) of from 300 to 6500 m² g⁻¹ and has at least macropores, mesopores and micropores, wherein the volume of the micropores (V_{mic}) is from 0.15 to 0.75 cm³ g⁻¹, preferably of activated carbon, of a metal-organic framework (MOF), of zeolite, of clay, of a porous organic polymer (POP), of hydrotalcite, of an organic-inorganic hybrid, of a porous metal oxide, of a lithium zirconate or of a mesoporous silica material (MSM), for neutralizing biotin interference in a binding assay for detection of an analyte in a sample, wherein the binding assay comprises contacting the sample at least with a biotinylated component and with a biotin-binding component.

## Revendications

1. Procédé de neutralisation d'une interférence de la biotine dans un test de fixation pour déceler un analyte dans un échantillon, dans lequel dans le test de fixation, on met l'échantillon en contact au moins avec un composant biotinilisé et avec un composant fixant la biotine, **caractérisé en ce que** l'on met l'échantillon avant ou pendant la mise en contact avec le composant biotinilisé et avec le composant fixant la biotine avec une substance adsorbante, dans lequel la substance adsorbante a une structure poreuse ayant une surface (S_{Bet}) d'ensemble de 300 à 6500 m² g⁻¹ et a au moins des macropores, des mésopores et des micropores, dans lequel le volume des micropores (V_{mic}) va de 0,15 à 0,75 cm³ g⁻¹.

2. Procédé suivant la revendication 1, dans lequel la substance adsorbante est du charbon actif, un composé (MOF) de structure organométallique, une zéolite, de l'argile, un polymère (POP) organique poreux, de l'hydrotalcite, un hybride organique-anorganique, un oxyde métallique poreux, un zirconate de lithium ou un matériau (MSM) de silice mésoporeux.

3. Procédé suivant l'une des revendications précédentes, dans lequel on met l'échantillon en contact avec la substance adsorbante en mélangeant en une suspension l'échantillon à la substance sous la forme d'une poudre de granulés ou d'un mélange de microfibres. .

4. Procédé suivant la revendication 3, dans lequel on sépare l'échantillon de la substance adsorbante avant la mise en contact venant ensuite avec le composant biotinilisé et avec le composant fixant la biotine.

5. Procédé suivant l'une des revendications 1 et 2, dans lequel on met l'échantillon en contact avec la substance adsorbante en mettant l'échantillon en contact avec une phase solide revêtue de la substance.

6. Procédé suivant la revendication 5, dans lequel la phase solide revêtue de la substance adsorbante est choisie dans le groupe d'un filtre, d'une surface d'un récipient à liquide, d'une pointe de pipette et de particules de latex, sépharose, agarose, matière plastique, verre, protéine, alginate, silicate ou métal.

7. Procédé suivant l'une des revendications précédentes, dans lequel on traite au préalable la substance adsorbante composée d'une ou de plusieurs protéines de blocage ou de peptides de blocage, de préférence d'une ou de plusieurs protéines de blocage du groupe de l'albumine, de la polygéline et de la caséine ou par du dextrane.

8. Procédé suivant l'une des revendications précédentes, dans lequel on prépare la substance adsorbante sous la forme de billes de substance revêtues de polymère d'une dimension de pore ayant de la perméabilité pour des molécules biologiques d'une masse inférieure à 40 kDa.

9. Procédé suivant l'une des revendications précédentes, dans lequel le composant fixant la biotine contient de l'avidine, de la streptavidine, de la tamavidine 1 ou 2, de la shwanavidine, de la rhizavidine, de la bradavidine, de la . burcavidine, de la zebavidine, de la xénavidine et/ou de la strongavidine fixant la biotine.

10. Procédé suivant l'une des revendications précédentes, dans lequel le composant fixant la biotine et/ou le composant biotinilisé contient un peptide, une protéine, un corps actif, un fragment de corps actif, un afimère ou un aptamère.

11. Procédé suivant l'une des revendications précédentes, dans lequel le composant biotinilisé contient un premier composant d'un système formant un signal et le composant fixant la biotine, un deuxième composant du système formant un signal et dans lequel le premier et le deuxième composants du système formant un signal coopèrent de manière à créer un signal détectable si le premier et le deuxième composants du système formant un signal sont mis ensemble à proximité dans l'espace.

12. Procédé suivant la revendication 11, dans lequel le premier composant du système formant un signal est un agent chemioluminescent et le deuxième composant du système formant un signal est un photosensibilisateur ou inversement.

13. Procédé suivant l'une des revendications précédentes, dans lequel l'échantillon est un échantillon de liquide corporel d'un homme ou d'un animal, de préférence du sang complet, du plasma sanguin, du sérum sanguin, de l'urine, de la liqueur ou du liquide lacrymal.

14. Utilisation d'une substance adsorbante, dans laquelle la substance a une structure poreuse ayant une surface (S_{Bet}) d'ensemble de 300 à 6500 m² g⁻¹ et comporte au moins des macropores, des mésopores et des micropores, dans lequel le . volume des micropores (V_{mic}) va de 0,15 à 0,75 cm³ g⁻¹, de préférence de charbon actif, d'un composé (MOF) de structure organométallique, d'une zéolithe, de l'argile, d'un polymère (POP) organique poreux, d'hydrotalcite, d'un hydrure organique-anorganique, d'un oxyde métallique poreux, d'un zirconate de lithium ou d'un matériau de silice (MSM) mésoporeux pour la neutralisation d'une interférence avec la biotine dans un test de fixation pour déceler un analyte dans un échantillon, dans lequel dans le test de fixation, l'échantillon est mis en contact au moins avec un composant biotinilisé et avec un composant fixant la biotine. .
